# EUROPÄISCHE PATENTSCHRIFT

(11) **EP 0 915 157 B1**
(45) Veröffentlichungstag und Bekanntmachung des Hinweises auf die Patenterteilung: **03.06.2009**
(21) Anmeldenummer: 98119012.7
(22) Anmeldetag: 08.10.1998
(51) Int. Cl.: C12N 15/12, C12N 15/70, C07K 14/745, C07K 14/81, C07K 16/36, A61K 38/36, G01N 33/86

(54) **Verfahren zur Herstellung von funktionalem rekombinantem Gewebsfaktor**
Method for the preparation of functional recombinant tissue factor
Procédé pour la préparation de facteur tissulaire fonctionel récombine

(30) Priorität: 07.11.1997 DE 19749259
(43) Veröffentlichungstag der Anmeldung: 12.05.1999
(73) Patentinhaber: Siemens Healthcare Diagnostics Products GmbH, 35041 Marburg (DE)
(72) Erfinder: Zander, Norbert, Dr., 35037 Marburg (DE); Wieczorek, Leszek, Dr., 35041 Marburg (DE)

(56) Entgegenhaltungen:
- EP-A- 0 200 417
- EP-A- 0 278 776
- WO-A-88/09817
- US-A- 5 639 635
- US-A- 5 658 790
- PABORSKY L R ET AL: "PURIFICATION OF RECOMBINANT HUMAN TISSUE FACTOR" BIOCHEMISTRY, AMERICAN CHEMICAL SOCIETY. EASTON, PA, US, Bd. 28, Nr. 20, 3. Oktober 1989 (1989-10-03), Seiten 8072-8077, XP000882409 ISSN: 0006-2960
- PATENT ABSTRACTS OF JAPAN vol. 1996, no. 06, 28. Juni 1996 (1996-06-28) & JP 08 038191 A (JAPAN ENERGY CORP), 13. Februar 1996 (1996-02-13)
- "Altered gene and E. coli strains for enhanced recombinant protein accumulation" CURRENT OPINION IN THERAPEUTIC PATENTS, Bd. 4, Nr. 2, 1994, Seiten 173-174, XP008022308

## Beschreibung

Die vorliegende Erfindung betrifft ein Verfahren zur Herstellung von funktionellem rekombinantem Gewebsfaktor in einem prokaryotischen Wirtsorganismus.

Kontakt von Blut mit Gewebeoberflächen führt zu einer deutlichen Beschleunigung der Gerinnung. Diese Beschleunigung beruht auf der spezifischen Wirkung eines Faktors aus dem Gewebe. Dieser "Gewebsfaktor" ist ein Protein, das mit Phospholipiden assoziiert sein muß, um prokoagulatorisch wirksam sein zu können. Die erste Aufreinigung des Proteins gelang 1980 aus Rinderhirn (Bach et al., J.Biol.Chem. 256(16), 8324 (1981), später gelang auch die Reinigung von menschlichem Gewebsfaktor (Broze et al., J.Biol.Chem. 260(20), 10917 (1985). Aminosäuresequenzen dieses Proteins wurden zum Design von Oligonukleotidsonden benutzt, mit deren Hilfe die Klonierung des Gens erfolgte. Das primäre Translationsprodukt ist ein Polypeptid aus 295 Aminosäuren. Die Oberflächendomäne mit der Rezeptordomäne nimmt die N-terminalen 219 Aminosäuren in Anspruch. Weiter C-terminal schließen sich eine 23 Aminosäuren lange Transmembrandomäne und ein 20 Aminosäuren langer cytoplasmatischer Anteil an (Edgington et al., Thromb. Haemostas. 66(1), 67 (1991). Das einzelne Cystein in der cytoplasmatischen Domäne kann als Akzeptor für einen Palmitat- oder Stearatrest dienen, der über eine Thioesterbindung verknüpft ist. Zwei intramolekulare Disulfidbrücken befinden sich in der extrazellulären Domäne; von diesen ist die C-terminale notwendig für die Bindung von Faktor Vlla. Die Oberflächendomäne ist über drei Threoninreste glykosyliert.

Nach dem heute verbreiteten Modell der Initiierung der Gerinnung führt etwa eine Gefäßläsion zum Kontakt von Blut mit Endothelzellen. Faktor VII oder Faktor Vlla aus Blutplasma bindet an den Gewebsfaktor-Rezeptor der Endothelzellen. In Gegenwart von Calcium und Phospholipid kommt es durch den Komplex an der Zelloberfläche zum Umsatz von Faktor X zu Faktor Xa. Dieser setzt seinerseits Prothrombin zu Thrombin, dieses Fibrinogen zu Fibrin um. Letztlich kommt es zur Bildung eines lokalen Gerinnsels.

Auf der Basis der klonierten menschlichen cDNA wurde eine Expression des Proteins in verschiedenen Systemen erreicht. Eine Überexpression in E.coli berichten z.B. Paborsky et al., Biochemistry 28, 8072 (1989). Auch in WO 88/09817 ist die Benutzung einer humanen cDNA in einem Verfahren zur Herstellung eines rekombinanten Gewebsfaktors in E. coli beschrieben.

Die Expression eukaryontischer Proteine in E.coli ist mit einer Reihe von prinzipiellen Problemen verknüpft. Eines dieser grundlegenden Probleme ist das Fehlen bakterieneigener Glykosylierungssysteme. Proteine, deren funktionelle Aktivität auf einer Glykosylierung beruht, lassen sich in aktiver Form nicht in E.coli exprimieren. Ein weiteres Problem ist die große Aktivität von zelleigenen Proteasen in E. coli (Maurizi et al., Experientia 48, 176 (1992), die die Stabilität der Translationsprodukte aus eingeschleusten Fremdgenen limitiert. Es sind verschiedene mutante E. coli-Stämme, wie z. B. Protease-defiziente Stämme bekannt, die eine Expression von heterologen Proteinen mit größerer Stabilität ermöglichen (siehe z. B. Altered gene and E. coli strains for enhanced recombinant protein accumulation, Exp. Opin. Ther. Patents (1994) 4 (2): 173-174).

Schließlich liegt ein grundsätzliches Problem der Expression von Gewebsfaktor in E.coli in der Tatsache, daß es sich bei diesem Protein um ein Membranprotein handelt. Für Membranproteine ist bekannt, daß ihre Expressionsrate deutlich geringer als die von löslichen Proteinen ist, vermutlich, weil das zelleigene Kompartiment, in das sich die überexprimierten Moleküle einlagern, die Membranen der Bakterienzellen, eine begrenzte Aufnahmekapazität besitzen. Zwar ist bereits beschrieben worden, daß diese Aufnahmekapazität durch die Ausbildung lamellarer Membranstrukturen (intrazelluläre Membraninvaginationen), ähnlich denen in Mitochondrien oder Chloroplasten erhöht werden kann, allerdings sind diese Strukturen bisher nur bei der Überexpression E. coli-spezifischer Membranproteine beobachtet worden, nicht jedoch bei heterologen eukaryotischen Proteinen ("exclusion bodies", von Meyenburg, K. et al. EMBO. Journal, Vol.3 1791-1797, 1984).,

Eine alternative Strategie ist deshalb die Expression eines mutierten Gewebsfaktor-Moleküls, dem die Transmembrandomäne fehlt. Dieser sogenannte 'lösliche' Gewebsfaktor wird im Cytoplasma der Bakterienzellen akkumuliert und läßt sich in größeren Mengen in E.coli exprimieren . Allerdings kann es in diesem System zu dem Problem kommen, daß Gewebsfaktor in Form sogenannter Einschlußkörper (inclusion bodies) in der E.coli-Zelle quasi-kristallin vorliegt. In diesem Fall müssen die Einschlußkörper durch die massive Anwendung chaotroper Agenzien solubilisiert werden und die so monomerisierten Proteine anschließend mit großem Aufwand und meist geringer Ausbeute wieder in eine aktive, renaturierte Konformation zurückgefaltet werden.

Für eine Anwendung in Prothrombinzeitreagenzien kommt der lösliche Gewebsfaktor jedoch grundsätzlich nicht in Frage, da ihm die Domäne für die Interaktion mit Phospholipiden fehlt. Vermieden werden könnten diese Schwierigkeiten nur dadurch, daß das native Protein schon während der Proteinbiosynthese in eine Lipidmembran integriert wird. Der besondere Vorteil des Verfahren läge darin, daß einerseits die Stabilität des Proteins durch die Membranintegration erhöht wird, andererseits dadurch auch die biologisch aktive Konformation stabilisiert wird. Ein weiterer Ansatz zur Überexpression des Gewebsfaktors ist die Verwendung einer Vielzahl von bekannten und erfolgreich eingesetzten Expressionssystemen, die für Produkte von Genfusionen kodieren (z.B. mit β-Galactosidase, MalE, Glutathion-Transferase, His-Tag). Diese sind jedoch für die Expression von biologisch aktivem Gewebsfaktor nicht geeignet. Zwar lassen sich bei Verwendung dieser Systeme durchaus Expressionsprodukte nachweisen, ebenso läßt sich auch die Expressionsleistung erhöhen, aber gleichzeitig ist dies mit einem vollständigen Funktionsverlust verbunden, wobei die Funktion auch durch die Anwendung aufwendiger Renaturierungsmethoden nicht wiederherzustellen ist.

Die verschiedenen angesprochenen Probleme der Überexpression in E. coli lassen sich durch Expression in einem eukaryontischen System umgehen. So kommen grundsätzlich Expression etwa in Hefe, in Insektenzellkultur mit Hilfe von Baculovirus als Vektor oder in kultivierten Säugerzellen, z.B. Ovarialzellen des Hamsters oder in menschlichen Zellinien (Paborsky et al., 1989) in Frage. Diese Systeme haben jedoch entscheidende Kostennachteile.

Ein Verfahren zur Herstellung von großen Mengen an vollständigem, biologisch aktivem, rekombinantem Gewebsfaktor aus E.coli mit großer Ausbeute und Reinheit ist bisher nicht bekannt.

Aufreinigungsstrategien von nativem und rekombinantem Gewebsfaktor sind in der Literatur beschrieben z. B. in Paborsky et al., 1989. Die Extraktion aus Zellen oder Gewebe erfolgt in der Regel mit Hilfe eines Detergenz, z.B. Desoxycholat oder Triton-X 100. Die Aufreinigungsstrategien enthalten verschiedene Chromatographieprozesse. Diese reichen von der Gelfiltration über lonenaustauschchromatographie, hydrophobe Interaktionschromatographie bis zu Affinitätschromatographieschritten. Als Affinitätsmaterialien sind Faktor und Antikörper gegen Gewebsfaktor (Paborsky et al., 1989) beschrieben. Den veröffentlichten Aufreinigungsverfahren ist gemeinsam, daß ihre Anwendbarkeit auf Fermentervolumen bis zu 1L eingeschränkt ist. Ein robustes Verfahren zur Herstellung von größeren Mengen reinen Gewebsfaktors im Technikumsmaßstab (10 - 100 L Fermentervolumen) ist bisher nicht beschrieben.

Gewebsfaktor wird z.B. verwendet in Prothrombinzeitreagenzien. Hierbei wird eine Blutplasmaprobe mit einem Überschuß von Gewebsfaktor in Gegenwart von Phospholipiden und Calcium zur Gerinnung gebracht. Diagnostisch relevant ist die Gerinnungszeit, die anhand geeigneter Kalibrationssysteme, etwa in Aktivitätswerte in % der Norm oder in Prothrombinratio-Werte, umgerechnet werden kann. Der Test dient zum Screening des extrinsischen Gerinnungssystems, etwa vor Operationen, der Überprüfung der Aktivität der einzelnen Faktoren des extrinsischen Systems der Gerinnung sowie der Therapiekontrolle bei oraler Antikoagulanzientherapie.

Neben dieser Standardanwendung sind auch andere diagnostische Anwendungen des Gewebsfaktors denkbar, etwa in einem Test für den 'Tissue Factor Pathway Inhibitor', in Neutralisationstests für Lupus-Antikoagulanzien oder als Verstärker- und Nachweissystem für immunologische Teste.

Eine therapeutische Anwendung wird zur Behandlung chronischer Blutungen diskutiert. Die Blutungsneigung kann hierbei angeboren (Hämophilie) oder erworben sein (Antikörper gegen Gerinnungsfaktoren, gestörte Synthese von Gerinnungsfaktoren in der Leber, Disseminierte Intravasale Gerinnung). In einem Tiermodell für Hämophilie konnte gezeigt werden, daß eine Injektion von rekombinantem Gewebsfaktor gut vertragen wurde und keine disseminierte intravasale Gerinnung eintrat. Durch diese Infusion konnte sogar für kurze Zeit das hämostatische Systems normalisiert werden (EP-A 0 278 776).

Der vorliegenden Erfindung lag also die Aufgabe zugrunde, ein robustes Verfahren zur Herstellung von größeren Mengen reinen Gewebsfaktors im Technikumsmaßstab (10 und mehr Liter Fermentervolumen), zu finden.

Überraschenderweise wurde nun gefunden, daß eine hohe Expressionsausbeute des vollständigen Gewebsfaktormoleküls in E. coli durch Verwendung eines Vektors erzielt werden kann, der eine Signalsequenz aus Bordetella pertussis enthält, die das Expressionsprodukt in den periplasmatischen Raum der Bakterienzelle dirigiert. Die bekannten Standardmedien und -verfahren zur Fermentation und Expression von rekombinanten Proteinen in E. coli führten nicht zum Erfolg. Es mußten wesentliche Umstellungen im Metabolismus der Wirtszelle induziert werden, damit einerseits die Expression des Thromboplastins eingeleitet wird und andererseits die Vitalität der Wirtszelle und die biologische Aktivität des Proteins erhalten bleibt. Für die erfolgreiche Umstellung des Metabolismus zur Herstellung des Gewebsfaktors während der Kultivierung des rekombinanten E. coli-Wirts ist erstaunlicherweise eine hohe Zelldichte wesentliche Voraussetzung. Eine hohe Zelldichte im Sinne der Erfindung ist eine Dichte von mindestens 50 g Zellen/l, bevorzugterweise 50 - 250 g Zellen/l, ganz bevorzugterweise 150 - 200 g Zellen/I . Dies entspricht etwa einer optischen Dichte bei 650 nm und 1 cm Schichtdicke von 50 - 110. Dies wird durch einen hohen Anteil an Vollmedium-Komponenten während der Kultivierung erreicht, wobei auch die Herkunft dieser Komponenten bedeutsam ist. Beispielsweise sind peptische oder tryptische Hydrolysate von tierischen Geweben (Pepton, Trypton, Luria-Broth) zwar für die Kultivierung des Organismus geeignet und liefern auch vergleichbare Biomassenerträge, aber diese Medien sind allein nicht geeignet, um die Voraussetzungen für eine Expression mit befriedigenden Ausbeuten an Proteinprodukt zu schaffen. Wichtig ist außerdem, daß eine weitere komplexe Wuchsstoffkomponente (Hefe-Extrakt) als Hauptbestandteil des Kultivierungsmediums zur Verfügung gestellt wird wie auch die Versorgung mit entsprechenden Spurenelementen durch eine separate Dotierung gewährleistet ist. Die wesentliche Voraussetzung für die Synthese des Genproduktes nach Derepression des Systems ist überraschenderweise, daß zu Beginn der Fermentation eine Kohlenstoffquelle (z.B. Glucose) zur Verfügung gestellt werden muß, die, nachdem sie aufgebraucht worden ist, zu einer dramatischen Änderung des E. coli-Metabolismus führt (C-Mangel, Diauxie-Effekte). Parallel hierzu fällt die respiratorische Leistung der Bakterienzellen ab, sichtbar an einer ebenso dramatischen Abnahme des Sauerstoffpartialdruckes (pO₂), obwohl die Zellen mit maximaler Luftversorgung kultiviert werden müssen. Dies allein führt noch nicht zu einer ergiebigen Proteinbiosynthese des Gewebsfaktors, sondern hierzu bedarf es eines genau definierten Mindestzeitraums, in dem die Zellen im Zustand des C-Hungers verbleiben. Erst wenn nach diesem Mindestzeitraum die Versorgung mit der C-Quelle wieder aufgenommen wird, kann auch die Synthese des humanen rekombinanten Gewebsfaktors effizient induziert werden. Wird dieser Zeitraum nicht eingehalten, findet keine oder höchstens eine um den Faktor 10 geringere Expression statt. Besonders überraschend in diesem Zusammenhang ist die Tatsache, daß die Bakterienzellen diese definierte Mindestverweilzeit im Zustand des Kohlenstoffmangels verbleiben müssen, um überhaupt den Induktionsvorgang (Derepression des Repressor-Proteins und Transkription des klonierten Gens) fortsetzen bzw. einleiten zu können. Für die Aufrechterhaltung der weiteren Synthese dagegen ist die kontinuierliche Zugabe der C-Quelle (z.B. Glucose) notwendig. Ferner wurde gefunden, daß die Produktstabilität sich durch Verwendung eines proteasedefizienten E.coli - Stamms verbessern läßt. Nach Aufschluß der Zellen läßt sich der Gewebsfaktor vollständig mit Detergenzien extrahieren. Die Abtrennung eines Großteils der begleitenden Wirtsproteine gelang überraschenderweise durch eine PEG-Fällung mit anschließender Tangentialflußfiltration, da PEG-Protein-Kopräzipitate normalerweise sehr empfindlich auf Scherkräfte reagieren und wieder auseinanderfallen. Die Abtrennung niedermolekularer Verunreinigungen wurde durch eine Diafiltration erreicht.

Um den Gewebsfaktor in diagnostischen oder therapeutischen Kompositionen einsetzen zu können, muß dieser in reiner und konzentrierter Form produziert werden. In diagnostischen Kompositionen sind vor allem Proteasen störend, die die Stabilität des Produkts limitieren. In therapeutischen Kompositionen können alle Fremdproteine aus E. coli potentiell zu nicht beabsichtigten Nebenwirkungen führen. Zur Aufreinigung von Proteinen dienen in der Regel chromatographische Verfahren. In Frage kommen etwa Gelchromatographie, hydrophobe Interaktionschromatographie, Ionenaustauschchromatographie oder Affinitätschromatographie. Bei Überexpression von heterologen cDNAs in E.coli reicht nicht selten ein einziger chromatographischer Schritt zur Reinigung aus. Die Aufreinigung von Gewebsfaktor im hier beschriebenen Verfahren hatte drei ungünstige Sachverhalte zu berücksichtigen:
1. Erstens ist der Gewebsfaktor in seiner exprimierten Form ein Membranprotein mit einer Transmembrandomäne. Dieses Protein ist in den standardmäßig zu Chromatographiezwecken verwendeten Puffersystemen nicht löslich. Es wurde gefunden, daß sich die nötigen Chromatographieschritte auch in Gegenwart eines Detergenz' durchführen lassen. Hierbei kommen verschiedene Klassen von Detergenzien in Frage. Bevorzugterweise werden aber nichtionische Detergenzien verwendet, um ionische Wechselwirkungen des Detergenz mit Liganden der verwendeten Matrizes auszuschließen. Verwendbar sind alle nichtionischen Detergenzien, bevorzugterweise Triton-X 100 oder Oktylglykosid, besonders bevorzugterweise Triton-X 100. Von entscheidender Bedeutung ist auch die Konzentration des verwendeten Detergenz'. Die kritische Mizellarkonzentration eines Detergenz gibt an, ab welcher Gesamtkonzentration sich spontan Mizellen aus Detergenzmolekülen bilden. Bei Triton X-100 liegt dieser Wert ionenstärkeabhängig zwischen 0.01 % und 0.06 %. Nach Möglichkeit sollte versucht werden, eine Konzentration unterhalb der kritischen Mizellarkonzentration einzustellen, um die Bildung von Detergenzmizellen zu unterbinden.
2. Die Menge des pro Zelle exprimierten Gewebsfaktors ist bei Membranproteinen eher gering. Anders als bei löslichen Proteinen, die nicht selten in so großer Menge produziert werden, daß sie innerhalb der Zellen in unlöslicher Form ausfallen, steht Membranproteinen, wenn sie in die richtigen Zellkompartimente geschleust werden, nur ein begrenzter Raum zur Verfügung. Im Falle des hier beschrieben Prozesses ist nach Extraktion nur < 1 % des im Extrakt enthaltenen Proteins das Produkt Gewebsfaktor. Über 99 % Begleitprotein muß also entfernt werden.
3. Weiterhin von Bedeutung ist der Erhalt von intaktem Protein im Extrakt beziehungsweise den Zwischenstufen des Aufreinigungsprozesses. Dies war nur durch den Einschluß von Proteaseinhibitoren in den Puffern zu erreichen. Setzt man keine Proteaseinhibitoren zu, ist in frischen Extrakten von E.coli ein massiver Proteinabbau schon unter kurzen Standzeiten beobachtbar. Für verschiedene Klassen von Proteasen sind Inhibitoren im Handel. Es wurde gefunden, daß die Verwendung von Inhibitoren gegen Serinproteasen den größten Erfolg bringen. Verwendbar sind alle Inhibitoren gegen Serinproteasen, bevorzugterweise Benzamidin und Antagosan. Inhibitoren gegen Mitglieder anderer Proteasefamilien (saure Proteasen, Cystein-Proteasen) oder gegen Amino- oder Carboxypeptidasen führten hingegen zu keiner verbesserten Stabilität des Gewebsfaktors.

Entscheidend für den Erfolg einer Aufreinigungsstrategie ist die Auswahl der chromatographischen Schritte und ihrer Reihenfolge. Überraschenderweise wurde gefunden, daß trotz der schwierigen Ausgangslage (geringe Konzentration des Produkts, Notwendigkeit des Einschlusses von Detergenzien, störende Proteasen) eine Aufreinigung bis zur Homogenität in nur zwei chromatographischen Schritten möglich war. Hierbei wurden eine lonenaustauschchromatographie und eine Affinitätschromatographie durchgeführt.

Für die Durchführung einer Ionenaustauschchromatographie kommen grundsätzlich Anionenaustauscher und Kationenaustauscher in Frage. Je nach pK-Wert der aufzureinigenden Proteine und nach dem eingestellten pH-Wert des Puffers binden verschiedene Proteine unterschiedlich stark an die Austauschgruppen und können durch steigende Mengen an Ionen eluiert werden. Bevorzugt im Rahmen dieser Erfindung sind starke Kationenaustauschergruppen, wie zum Beispiel Mono-S, oder starke Anionenaustauschergruppen, wie z.B. DEAE, QAE oder Mono-Q. Besonders bevorzugt als Anionentauscher ist DEAE, da der Gewebsfaktor bei physiologischen pH-Werten (ca. 8) bindet. Ionenaustauschergruppen können an verschiedene Trägermaterialien gebunden werden. Möglich ist z.B. die Verwendung von Sepharose, Agarose oder Cellulose. Bevorzugterweise wurde quervernetzte Cellulose verwendet, da dieses Material eine sehr gute Druckstabilität aufweist.

Die bevorzugte Durchführung der Ionenaustauschchromatographie ist im folgenden beschrieben, ohne andere Durchführungsmöglichkeiten ausschließen zu wollen. Aus E.coli extrahierter Gewebsfaktor wurde wie oben beschrieben extrahiert und durch PEG-Fällung und Diafiltration vorgereinigt. Dieser Extrakt wurde mit Proteaseinhibitoren versetzt und auf die Ionenaustauschersäule aufgetragen. Die besonders bevorzugterweise verwendete Säule besteht aus einer modifizierten, quervernetzten Cellulose mit dem starken Anionentauscher DEAE. Die effektive Konzentration an Triton-X 100 an dieser Stelle im Prozeß ist ca. 1 %, also weit oberhalb der kritischen Mizellenkonzentration. Es ist also damit zu rechnen, daß sich gemischte Mizellen aus Detergenzmolekülen, E.coli-Lipiden und Membranproteinen bilden. Überraschenderweise wurde gefunden, daß Gewebsfaktor trotz dieser Tatsache vollständig an das Säulenmaterial bindet. Die Elution des Gewebsfaktors erfolgte mit steigenden Mengen Salz, bevorzugterweise Natriumsalzen, besonders bevorzugterweise Natriumchlorid. Die zu vollständiger Elution nötige Konzentration ist < 0.1 M. Die erzielte Ausbeute der Ionenaustauschchromatographie ist in der Regel > 80 %, die erzielte Aufreinigung ist ca. 3-fach.

Für einen zweiten Aufreinigungsschritt fiel die Wahl auf ein affinitätschromatographisches Verfahren. Das Eluat der Ionenaustauschchromatographie enthält noch immer < 3 % Gewebsfaktor bezogen auf Gesamtprotein, so daß die Wahl eines maximal spezifischen Schritts zur Hochreinigung sinnvoll ist. Als Affinitätsliganden kommen im Prinzip Antikörper, Farbstoffe oder physiologische Liganden in Frage. Bevorzugterweise wurden Antikörper gegen Gewebsfaktor, besonders bevorzugterweise monoklonale Antikörper, verwendet. Der Affinitätsligand wird, in der Regel kovalent, an ein Trägermaterial gekoppelt. Möglich sind erneut etwa Agarose, Sepharose oder Cellulose. Im Fall der hier beschriebenen Erfindung wurde bevorzugterweise Sepharose verwendet, die bereits voraktiviert war. Die Kopplung des Antikörpers erfolgte mittels der Epoxy-Chemie. Das Eluat der Ionenaustauschchromatographie wurde auf die Antikörper-/Sepharosesäule aufgetragen. Ein Waschschritt erfolgte mit einem proteaseinhibitorhaltigem Puffer, der Triton X-100 in einer unterhalb der kritischen Mizellarkonzentration liegenden Konzentration enthielt. Zur Elution muß der sich bildende Antigen-Antikörper-Komplex aufgelöst werden. Hierzu können etwa Salze, chaotrope Agenzien oder Säure verwendet werden. Im Rahmen dieser Erfindung wurde bevorzugterweise ein Glyzinpuffer, besonders bevorzugterweise bei pH 2, verwendet. Der eluierte Gewebsfaktor wurde neutralisiert.

Überraschenderweise wurde gefunden, daß der so aufgereinigte Gewebsfaktor gelelektrophoretisch rein ist. Verunreinigungen in nennenswerter Konzentration sind nicht mehr feststellbar. Der Gewebsfaktor kann im eingefrorenen Zustand ohne Zusatz von Stabilisatoren viele Monate lang gelagert werden. Nach Ankonzentrierung kann der Gewebsfaktor zu verschiedenen Zwecken eingesetzt werden, zum Beispiel zur Herstellung eines Prothrombinzeitreagenz, als Standard für den Nachweis von Gewebsfaktor in biologischen Proben oder auch als Bestandteil therapeutisch wirksamer Kompositionen zum Zwecke der Normalisierung des Gerinnungssystems in vivo.

Die folgenden Beispiele sollen die Erfindung erläutern.

### Beispiele

### Beispiel 1:

### Herstellung des rekombinanten Gewebsfaktors in kleinem Maßstab (10 L-Fermentation)

Vorkultur: 600 ml Luria-Broth-Medium (LB-Medium) mit 50 µg Ampicillin/ml werden mit dem transformierten E. coli-Stamm inoculiert und 16 Stunden bei 37°C in Schüttelinkubator kultiviert. Nach Zugabe von weiteren 400 ml LB-Medium (50 µg Ampicillin/ml) wird die Kultur nochmals 3,5 bis 4,5 Stunden inkubiert. 800 ml dieser Schüttelkolbenkultur dienen als Inoculum für den Fermenter.

10 L-Fermenter: Für die Fermentation werden 5 L-Medium vorgelegt mit folgender Zusammensetzung: Hefeextrakt 324 g; Tryptone 84 g; Natriumchlorid 42 g; Ammoniumsulfat 8,8 g; Glucose 125 g; Spurenelementlösung 8,4 ml (Zusammensetzung: 32 mM Borsäure; 0.64 mM Ammoniummolybdat; 0.64 mM Kupfer-II-sulfat; 2.4 mM Kaliumjodid; 12 mM Mangan-II-sulfat; 5.56 mM Zinksulfat);

Mineralmedium 670 ml (Zusammensetzung: 27.5 mM di-Natriumhydrogenphosphat; 10.8 mM Natriumdihydrogenphosphat;10.4 mM Magnesiumsulfat; 16.8 mM Kaliumchlorid; 25 mM Citronensäure; 0.8 mM Eisen-III-sulfat).

Das Medium wird auf 100 µg Ampicillin/ml und 1 µg Thiamin/ml eingestellt. Der Fermenter wird mit 800 ml der Vorkultur beimpft und bei 37°C und maximaler Belüftung unter Einregelung des pH-Wertes auf 7.0 die Bakterien vermehrt. 7.5 Stunden nach Fermentationsstart werden 2.0 bis 2.6 L einer 50%igen Glucose-Lösung bis zum Ende der Fermentation zugepumpt (100 ml/Stunde). 19.5 bis 20.5 Stunden nach Fermentationsbeginn wird die Expression der rekombinanten Gewebsfaktors durch die Zugabe von IPTG (Endkonzentration 1 bis 5 mM) eingeleitet und weitere 5.5 bis 6.5 Stunden inkubiert. Anschließend wird die Biomasse durch Zentrifugation geerntet. Ausbeute ca. 1 bis 1,5 kg.

Aufschluß: Die gesamte Biomasse wird in 4 L 20 mM Tris-HCl (pH7.5), 5 mM EDTA, 1 mM Benzamidin, 0.2 mM Phenylmethylsulfonylfluorid resuspendiert, in einem Zellhomogenisator (APV-Gaulin) homogenisiert und der Zellextrakt nach Einstellung auf 1 % Triton X-100 eine Stunde bei 0° bis 8°C gerührt. Anschließend wird 2% Polyethylenglykol 600 zugegeben, eine weitere Stunde gerührt und das Zelllysat durch Tangentialflußmikrofiltration (0.2 µm-Filter) klar filtriert, wobei unter Beibehaltung des retentatseitigen Volumens ein bis zwei Aufschlußvolumina zur Auswaschung der Zelltrümmer zusätzlich durchgesetzt wurden (Puffer: 20 mM Tris-HCl (pH7.5), 5 mM EDTA, 1 mM Benzamidin, 0.2 mM Phenylmethylsulfonylfluorid, 0.1 % Triton X-100). Das Filtrat wird durch Tangentialultrafiltration (10.000 kd) auf das Extraktausgangsvolumen einkonzentriert und mit mindestens 5 Volumen Puffer (20 mM Tris-HCl (pH7.5), 5 mM EDTA, 1 mM Benzamidin, 0.2 mM Phenylmethylsulfonylfluorid) diafiltriert. Ausbeute zwischen 16 und 50 mg Thromboplastin je Liter Extrakt.

### Beispiel 2:

### Herstellung des rekombinanten Gewebsfaktors in großem Maßstab (100 L-Fermentation)

Vorkultur: 8 L Luri-Broth-Medium (LB-Medium) mit 50 µg Ampicillin/ml werden mit dem transformierten E. coli-Stamm inoculiert und 16 bis 20 Stunden bei 37°C in einem 10 L-Fermenter kultiviert. 8 L dieser Schüttelkolbenkultur dienen als Inoculum für den Fermenter.
100 L-Fermenter: Für die Fermentation werden 50 L-Medium, das folgende Komponenten und Mengen enthält, vorgelegt: Hefeextrakt 3240 g; Tryptone 840 g; Natriumchlorid 420 g; Ammoniumsulfat 88 g: Glucose 1250 g; Spurenelementlösung 84 ml (Zusammensetzung:.s.o); Mineralmedium 6.7 L (Zusammensetzung:.s.o.). Das Medium wird auf 100 µg Ampicillin/ml und 1 µg Thiamin/ml eingestellt.

Der Fermenter wird mit 8 L der Vorkultur beimpft und bei 37°C und maximaler Belüftung unter Einregelung des pH-Wertes auf 7.0 die Bakterien vermehrt. 7.5 Stunden nach Fermentationsstart werden 20 bis 26 L einer 50%igen Glucose-Lösung bis zum Ende der Fermentation zugepumpt (1 L/Stunde). 19.5 bis 20.5 Stunden nach Fermentationsbeginn wird die Expression der rekombinanten Gewebsfaktors durch die Zugabe von IPTG (Endkonzentration 1 bis 5 mM) eingeleitet und weitere 5.5 bis 6.5 Stunden inkubiert. Anschließend wird die Biomasse durch Zentrifugation geerntet. Ausbeute ca. 10 bis 15 kg.

Aufschluß: Die gesamte Biomasse wird in 40 L 20 mM Tris-HCl (pH7.5), 5 mM EDTA, 1 mM Benzamidin, 0.2 mM Phenylmethylsulfonylfluorid resuspendiert, in einem Zellhomogenisator (APV-Gaulin) homogenisiert und der Zellextrakt nach Einstellung auf 1 % Triton X-100 eine Stunde bei 0° bis 8°C gerührt. Anschließend wird 2% Polyethylenglykol 600 zugegeben, eine weitere Stunde gerührt und das Zelllysat durch Tangentialflußmikrofiltration (0.2 µm-Filter) klar filtriert, wobei unter Beibehaltung des retentatseitigen Volumens ein bis zwei Aufschlußvolumina zur Auswaschung der Zelltrümmer zusätzlich durchgesetzt wurden (Puffer: 20 mM Tris-HCl (pH7.5), 5 mM EDTA, 1 mM Benzamidin, 0.2 mM Phenylmethylsulfonylfluorid, 0.1 % Triton X-100). Das Filtrat wird durch Tangentialultrafiltration (10.000 kd) auf das Extraktausgangsvolumen einkonzentriert und mit mindestens 5 Volumen Puffer (20 mM Tris-HCl (pH7.5), 5 mM EDTA, 1 mM Benzamidin, 0.2 mM Phenylmethylsulfonylfluorid) diafiltriert.
Ausbeute zwischen 16 bis 50 mg Thromboplastin je Liter Extrakt.

### Beispiel 3:

### Aufreinigung von Gewebsfaktor durch lonenaustauscherchromato-graphie

Der detergenzhaltige Extrakt der E.coli-Zellen wird 1+1 mit destilliertem Wasser verdünnt. Ist die Leitfähigkeit größer als 3 mS/cm, wird mit Aqua dest. solange nachverdünnt, bis dieser Wert unterschritten ist. Anschließend wird der Ansatz über eine Satrobran/Mini-Filterkerze (0.45 µm / 0.2 µm) filtriert.

Der Ionentauscher Cellufine - A 500 (Amicon) wird als vorgequollenes Gel geliefert. Eine Menge, die einem späteren Gelbettvolumen von 5 L entspricht, wird in eine Vantage- S 250- Chromatographiesäule (Amicon) eingefüllt. Vor dem Lauf wird das Gelmaterial mit einigen Gelvolumina Grundpuffer (10 mM Tris, 1 mM Benzamidin, 5 KIE/mL Antagosan, 0.1 mM EDTA, 0.1 % Natriumazid, 0.1 % Triton-X 100, pH 8.0) äquilibriert.

Der Säulenlauf erfolgt automatisch auf einer FPLC-Anlage mit automatischer Gradientenbildung und einer Pumpgeschwindigkeit von 5 L pro Stunde. Nacheinander werden folgende Flüssigkeiten über die Säule gepumpt:
1. 10 L verdünnter E.coli- Extrakt
2. 10 L Grundpuffer
3. 10 L Grundpuffer, aufgestockt mit 100 mM NaCl
4. 10 L Grundpuffer, aufgestockt mit 200 mM NaCl
5. 10 L Grundpuffer, aufgestockt mit 1 M NaCl
6. 10 L 0.2 N NaOH, 1 M NaCl
7. 10 L 0.2 N HCl, 1M NaCl
8. 10 L 0.2 M Tris, pH 8
9. 10 L Grundpuffer

Über den gesamten Lauf werden pH-Wert, Optische Dichte bei 280 nm und Ionenstärke aufgezeichnet. Die Elution des Gewebsfaktors erfolgt durch einen Stufengradienten mit steigender Salzkonzentration. Die drei Fraktionen, die den Puffern mit Konzentrationen von 0.1 M, 0.2 M und 1 M NaCl entsprechen, werden automatisch gesammelt. Die Regenerierung der Säule erfolgt durch je einen Laugen-, Säuren- und Neutralisierungsschritt.
Von allen Aufreinigungsschritten werden Proben genommen und in einem ELISA für Gewebsfaktor auf ihren Gehalt überprüft. Die folgende Tabelle zeigt die Ergebnisse eines Aufreinigungslaufs.

| Stufe | Vol | Gewebsfaktor | Gewebsfaktor | Gewebsfaktor |
|---|---|---|---|---|
| | [L] | [mg/L] | [mg] | [%] |
| Extrakt | 10 | 16.4 | 164 | 100 |
| Durchlauf | 20 | < 0.1 | < 2 | < 1 |
| Eluat 0.1 M NaCl | 10 | 13.6 | 136 | 83 |
| Eluat 0.2 M NaCl | 10 | 3.6 | 36 | 22 |
| Eluat 1 M NaCl | 10 | < 0.1 | < 1 | < 1 |
| Regenerat | 40 | 0 | 0 | 0 |

Der Gewebsfaktor findet sich nahezu ausschließlich in der Eluatfraktion mit 0.1 M NaCl. Die Ausbeute ist > 80%. Diese Fraktion wird zur weiteren Aufreinigung in der Affinitätschromatographie (siehe folgendes Beispiel) verwendet.

### Beispiel 4:

### Aufreinigung von Gewebsfaktor durch Affinitätschromatographie

Ein monoklonaler Antikörper gegen Gewebsfaktor wird an Epoxy-aktivierte Sepharose 6B (Pharmacia) in einer Konzentration von 1 g Antikörper pro 100 g festes Trägermaterial mit Hilfe von Standardmethoden nach Angaben des Herstellers kovalent gekoppelt. Das Material wird in eine Chromatographiesäule eingefüllt und mit Grundpuffer (40 mM HEPES, 1 mM Benzamidin, 0.1 mM EDTA, 5 KIE/mL Antagosan, 0.05 % Triton-X 100, pH 7.5) äquilibriert.

Die gewebsfaktorhaltige Eluatfraktion der Ionenaustauschchromatographie wird als Auftrag verwendet.

Der Säulenlauf erfolgt automatisch auf einer FPLC-Anlage mit automatischer Gradientenbildung und einer Pumpgeschwindigkeit von 1 L pro Stunde. Nacheinander werden folgende Flüssigkeiten über die Säule gepumpt:

| | |
|---|---|
| 1. | 10 L gewebsfaktorhaltige Eluatfraktion der lonenaustauschchromatographie |
| 2. | 2 L Grundpuffer |
| 3. | 1 L Elutionspuffer (50 mM Glyzin, 0.1 mM EDTA, 0.05 % Triton- X 100, pH 2.0) |
| 4. | 1 L Grundpuffer |
| 5. | 1 L 1 N Essigsäure |
| 6. | 2 L Grundpuffer |

Über den gesamten Lauf werden pH-Wert, Optische Dichte bei 280 nm und Ionenstärke aufgezeichnet. Die Elution des Gewebsfaktors erfolgt durch einen pH-Sprung von 8.0 auf 2.0. Die Sammlung des Eluats beginnt automatisch, sobald der pH-Wert einen Grenzwert von 6.5 unterschreitet, und endet nach Umschalten auf Grundpuffer und dem Wiederansteigen des pH-Werts auf über 6.8.

Das Eluat wird in einer Filtron-Konzentrierungsanlage (Minisette) über eine Filterschicht von 10 kDa Ausschlußgröße auf minimales Volumen (ca. 40 mL) ankonzentriert. Anschließend wird das Konzentrat über einen 0.2 µm Aufgußfilter filtriert und bei -70°C eingefroren

Von allen Aufreinigungsschritten werden Proben genommen und in einem ELISA für Gewebsfaktor auf ihren Gehalt überprüft. Die folgende Tabelle zeigt die Ergebnisse eines Aufreinigungslaufs.

| Stufe | Vol [L] | Gewebsfaktor [mg/L] | Gewebsfaktor [mg] | Gewebsfaktor [%] |
|---|---|---|---|---|
| Auftrag | 10 | 13.6 | 136 | 100 |
| Durchlauf | 12 | < 0.1 | < 2 | < 1 |
| Eluat | 1 | 123 | 123 | 90 |
| Konzentrat | 0.04 | 2450 | 98 | 72 |
| Regenerat | 4 | < 0.1 | < 4 | < 2 |

Der Gewebsfaktor findet sich ausschließlich im Eluat bzw. im Konzentrat. Die Ausbeute ist > 80 %. Die Anreicherung in diesem Schritt bezogen auf den Gesamtproteingehalt ist ca. 500-fach.

### Beispiel 5:

### Verwendung von reinem Gewebsfaktor in einem Prothrombinzeitreagenz

Gewebsfaktor wird in E.coli exprimiert. Die Zellen werden aufgeschlossen und mit Detergenz extrahiert. Gewebsfaktor wird aus dem Extrakt über lonenaustausch-chromatographie und Affinitätschromatographie bis zur Homogenität aufgereinigt.

Zur Relipidisierung werden gemischt:

| | |
|---|---|
| 125 µl | 10 % Sojabohnen-Phospholipide (Phospholipon 25 P von Nattermann) in 50 mM Glyzin pH 3 |
| 10 µl | 20 % Triton- X 100 |
| 50 µl | reiner Gewebsfaktor (2 mg/ml) |
| 15 µl | Aqua dest. |

Es wird 2 Stunden bei 15°C - 25°C unter Mischen inkubiert. Anschließend wird der Ansatz mit 100 ml Reagenzpuffer, bestehend aus 50 mM HEPES pH 7.5, 12 mM Calciumchlorid, 0.1 % Natriumazid und 0.5 % Polygelin verdünnt. Das Prothrombinzeitreagenz ist hiermit verwendungsfertig.

Die Gerinnungszeit von Plasmen wird wie folgt bestimmt. Am Behring Fibrintimer A werden 50 µl Plasma mit 100 µl auf 37 °C vorgewärmtem Prothrombinzeitreagenz vermischt. Der Gerinnungszeitpunkt wird turbidimetrisch ermittelt. Die folgende Tabelle zeigt einige auf diese Weise ermittelte Gerinnungszeiten im Vergleich zu einem konventionellen Prothrombinzeitreagenz, Thromborel S von Behring Diagnostics. Dieses Reagenz beruht auf nativem Gewebsfaktor aus Humanplazenta.

| Plasma | Verdünnung mit isotonischer NaCl-Lösung | Gerinnungszeit mit Thromborel S [s] | Gerinnungszeit mit Prothrombinzeitreagenz gemäß diesem Beispiel [s] |
|---|---|---|---|
| Lyophilisierter Normalplasmapool | 1 +0 | 12.1 | 11.3 |
| Lyophilisierter Normalplasmapool | 1+1 | 15.5 | 16.9 |
| Lyophilisierter Normalplasmapool | 1 +2 | 19 | 22 |
| Lyophilisierter Normalplasmapool | 1 +3 | 22 | 28.4 |
| Lyophilisierter Normalplasmapool | 1 +4 | 24.3 | 33 |
| Lyophilisierter Normalplasmapool | 1+5 | 27.5 | 38.2 |
| Lyophilisierter Pool von Patienten unter oraler Antikoagulation #1 | 1 +0 | 36.4 | 42.1 |

Die mit dem wie oben hergestellten Prothrombinzeitreagenz ermittelten Gerinnungszeiten sind ähnlich den mit Thromborel S ermittelten Gerinnungszeiten. Die Empfindlichkeit des laut Beispiel hergestellten Prothrombinzeitreagenzes gegenüber Faktorenmängeln ist größer als die Empfindlichkeit des Referenzreagenzes.

### Beispiel 6:

### Verwendung von reinem Gewebsfaktor als Standard für einen Nachweis von Gewebsfaktor

Gewebsfaktor wird in E.coli exprimiert. Die Zellen werden aufgeschlossen und mit Detergenz extrahiert. Gewebsfaktor wird aus dem Extrakt über lonenaustauschchromatographie und Affinitätschromatographie bis zur Homogenität aufgereinigt. Der Proteingehalt des reinen Gewebsfaktors wird bestimmt.

Für einen ELISA-Aufbau wird eine Testplatte mit 1.5 µg monoklonalem Antikörper gegen Gewebsfaktor pro Reaktionsplatz beschichtet. An einen polyklonalen Antikörper gegen Gewebsfaktor wird das Enzym Meerrettich-Peroxidase kovalent gekoppelt.

Reiner Gewebsfaktor (2 mg/ml Protein) wird stufenweise mit einem Verdünnungspuffer (50 mM HEPES, 13 mM Calciumchlorid, 65 mM Glyzin, 1 % Triton- X 100, 0.1 % Natriumazid, pH 7.5) verdünnt.
Zu einem jeden Reaktionsplatz einer Testplatte werden gegeben:

| | |
|---|---|
| 50 µl | verdünnter Gewebsfaktor als Standard (oder ggf. verdünnte Probe) |
| 50 µl | Probenpuffer |
| | (100 mM Tris, 10 mM EDTA, 1 % Triton-X 100, 0.1 % Natriumazid, pH 7.5) |

Die Platte wird mit einer Klebefolie abgedeckt und 15-20 Minuten bei 37 °C im Wasserbad inkubiert. Die Platte wird drei Mal mit 100 µl 'Waschlösung für Enzygnost' (Behring Diagnostics) gewaschen.

Anschließend werden jeweils 100 µl Peroxidase-Konjugat des polyklonalen Antiserums zugegeben. Die Platte wird erneut mit einer Klebefolie abgedeckt und 15-20 Minuten bei 37 °C im Wasserbad inkubiert.

In dieser Zeit wird pro Testplatte eine Abfüllung 'Chromogen für Enzygnost' (Behring Diagnostics) in einer Abfüllung 'Puffer/Substrat-POD für Enzygnost' (Behring Diagnostics gelöst.

Die Platte wird drei Mal mit je 100 µl 'Waschlösung für Enzygnost' (Behring Diagnostics) gewaschen.

In jede Vertiefung werden nun 100 µl 'Chromogen für Enzygnost' pipettiert. Die Platte wird 5 Minuten lang bei Raumtemperatur im Dunkeln inkubiert. Anschließend werden in jede Vertiefung werden 100 µl 'Stoplösung für Enzygnost' (Behring Diagnostics) pipettiert.

Die photometrische Auswertung bei 492 nm erfolgt spätestens eine Stunde nach dem letzten Pipettierschritt.

Die folgende Tabelle zeigt die Ergebnisse:

| Probe | Verdünnung | Extinktion bei 492 nm | Gewebsfaktor [mg/L] |
|---|---|---|---|
| Gewebsfaktor 2 mg/mL | 1:1 000 | 0.776 | 2.000 |
| Gewebsfaktor 2 mg/mL | 1: 2 000 | 0.676 | 1.000 |
| Gewebsfaktor 2 mg/mL | 1: 4 000 | 0.475 | 0.500 |
| Gewebsfaktor 2 mg/mL | 1: 8 000 | 0.300 | 0.250 |
| Gewebsfaktor 2 mg/mL | 1 : 16 000 | 0.203 | 0.125 |
| Gewebsfaktor 2 mglmL | 1 : 32 000 | 0.122 | 0.063 |
| Gewebsfaktor 2 mg/mL | 1 : 64 000 | 0.069 | 0.032 |
| Plazentaextrakt | | 0.295 | 0.250 |
| E. coli-Extrakt | 1 : 100 | 0.205 | 12.500 |
| Reiner Gewebsfaktor (Konzentrat) | 1 : 1000 | 0.717 | 2 152.000 |

Der aus E.coli aufgereinigte rekombinante Gewebsfaktor kann als Standard für den hier beschriebenen ELISA-Aufbau verwendet werden. Der Nachweis ist empfindlich über einen Bereich von zwei Größenordnungen. Aus einem Auftrag der Werte für die Standardverdünnungen (Extinktionswerte gegen den Gehalt in doppelt-logarithmischer Form) lassen sich die Gehalte von Proben bestimmen.

## Patentansprüche

1. Verfahren zur Herstellung von funktionalem rekombinantem Gewebsfaktor in Escherichia coli, **dadurch gekennzeichnet, daß**
a) zur Kultivierung der prokaryotischen Organismen ein Medium eingesetzt wird, das mindestens die folgenden Bestandteile enthält: Hefe-Extrakt und peptische oder tryptische Hydrolysate von tierischen Geweben, und
b) die Derepression des rekombinanten Gens in der stationären Wuchsphase der Bakterien erfolgt in Anwesenheit einer Kohlenstoffquelle, wobei diese Kohlenstoffquelle im Verlauf der Derepressionsanlaufphase verbraucht wird und die Zellen anschließend einen definierten Zeitraum, mindestens aber 1.5 Stunden, im Zustand des Kohlenstoffmangels verbleiben, und
c) die Zellen anschließend bei maximalem Sauerstoffeintrag kontinuierlich während der Expression mit der Kohlenstoff-Quelle versorgt werden.

2. Verfahren nach Anspruch 1 , **dadurch gekennzeichnet, daß** mindestens eine Protease dieses Wirtsorganismus genetisch ausgeschaltet ist.

3. Verfahren nach Anspruch 1 , **dadurch gekennzeichnet, daß** ein Plasmidvektor benutzt wird, der die genetische Information der humanen c-DNA enthält.

4. Verfahren nach Anspruch 3 , **dadurch gekennzeichnet, daß** dieser Plasmidvektor einen induzierbaren oder dereprimierbaren Promotor oder eine spezifische Signalsequenz enthält, die das primäre Translationsprodukt in ein primäres Zellkompartiment transportiert.

5. Verfahren nach Anspruch 1 **dadurch gekennzeichnet, daß** zur Kultivierung der prokaryotischen Organismen ein Medium eingesetzt wird, daß mindestens die folgenden Bestandteile enthält: Hefe-Extrakt, Trypton-Extrakt, NaCl, Malzextrakt und Puffersalze.

6. Verfahren nach Anspruch 5 , **dadurch gekennzeichnet, daß** die so gewonnenen Zellen durch Homogenisierung aufgeschlossen werden und anschließend in Gegenwart eines Detergenz inkubiert werden.

7. Verfahren nach Anspruch 6 , **dadurch gekennzeichnet, daß** der detergenzhaltige Extrakt mit Polyäthylenglykol versetzt wird, vorzugsweise PEG 600, und damit auf eine Konzentration von 0 bis 40 %, vorzugsweise ein % eingestellt wird und nach Inkubation bei 0 bis 25 ° C, vorzugsweise 4 ° C, über 0 bis 6 Stunden, vorzugsweise eine Stunde, klarfiltriert, vorzugsweise durch Tangentialflußfiftration, wird.

8. Verfahren nach Anspruch 7 , **dadurch gekennzeichnet, daß** das geklärte Filtrat durch Diafiltration über Membranen, Molekulargewichtsausschlußgröße 5.000 bis 50.000 Dalton, vorzugsweise 10.000 Dalton, weiter prozessiert wird.

9. Verfahren nach Anspruch 8, **dadurch gekennzeichnet, daß** der Gewebsfaktor aus dem Retentat der Diafiltration weiter aufgereinigt wird.

10. Verfahren nach Anspruch 9, **dadurch gekennzeichnet, daß** ein mehrstufiges chromatographisches Aufreinigungsverfahren verwendet wird.

11. Verfahren nach Anspruch 10, **dadurch gekennzeichnet, daß** einer der chromatographischen Schritte eine Ionenaustauschchromatographie an einem Kationenaustauscher oder einem Anionenaustauscher ist.

12. Verfahren nach Anspruch 11, **dadurch gekennzeichnet, daß** eine starke Anionenaustauschgruppe, vorzugsweise DEAE, gekoppelt an ein druckfestes Trägermaterial, vorzugsweise Cellulose, verwendet wird.

13. Verfahren nach Anspruch 12, **dadurch gekennzeichnet, daß** dem Puffer Inhibitoren gegen Proteasen zugesetzt sind.

14. Verfahren nach Anspruch 13, **dadurch gekennzeichnet, daß** ein affinitätschromatographischer Schritt eingeschlossen ist.

15. Verfahren nach Anspruch 14, **dadurch gekennzeichnet, daß** als Affinitätsligand ein Antikörper gegen Gewebsfaktor oder ein physiologischer Ligand von Gewebsfaktor verwendet wird.

16. Verfahren nach Anspruch 15, **dadurch gekennzeichnet, daß** ein polyklonaler oder monoklonaler Antikörper gegen Gewebsfaktor an ein druckfestes Säulenmaterial, bevorzugterweise Sepharose, kovalent gekoppelt wird.

17. Verfahren nach Anspruch 16, **dadurch gekennzeichnet, daß** der Gewebsfaktor durch Verwendung von Salz, Puffer mit einem bestimmten pH-Wert oder chaotropen Agenzien, bevorzugterweise durch verdünnten Glyzinpuffer mit saurem pH, besonders bevorzugterweise mit 50 mM Glyzin pH 2, vom Affinitätsliganden abgetrennt wird.

18. Verfahren nach Anspruch 10, **dadurch gekennzeichnet, daß** der chromatographisch aufgereinigte Gewebsfaktor über ein Diafiltrationsverfahren bis auf eine Konzentration von > 1 g/l ankonzentriert wird.

## Claims

1. A process for preparing functional recombinant tissue factor, in Escherichia coli, which comprises
a) for culturing the prokaryotic organisms, employing a medium which at least contains the following constituents: yeast extract and peptic or tryptic hydrolysates of animal tissues, and
b) the recombinant gene in the stationary growth phase of the bacteria being derepressed in the presence of a carbon source, with this carbon source being consumed during the course of the derepression start-up phase and the cells then remaining for a defined period, but at least 1.5 hours, in a state of carbon deficiency, and
c) the cells subsequently being supplied continuously, during the expression, with the carbon source, while oxygen is being provided at a maximum rate.

2. The process as claimed in claim 1, wherein at least one protease of this host organism is eliminated genetically.

3. The process as claimed in claim 1, wherein use is made of a plasmid vector which contains the genetic information of the human cDNA.

4. The process as claimed in claim 3, wherein this plasmid vector contains an inducible or derepressible promoter or a specific signal sequence which transports the primary translation product into a primary cell compartment.

5. The process as claimed in claim 1, wherein a medium which at least contains the following constituents: yeast extract, tryptone extract, NaCl, malt extract and buffer salts is employed for culturing the prokaryotic organisms.

6. The process as claimed in claim 5, wherein the cells which have been obtained in this way are disrupted by homogenization and then incubated in the presence of a detergent.

7. The process as claimed in claim 6, wherein the detergent-containing extract is treated with polyethylene glycol, preferably PEG 600, and thereby brought to a concentration of from 0 to 40%, preferably of one %, and, after having been incubated at from 0 to 25°C, preferably at 9°C, for from 0 to 6 hours, preferably for one hour, is clarified by filtration, preferably by means of tangential flow filtration.

8. The process as claimed in claim 7, wherein the clarified filtrate is further processed by means of diafiltration through membranes having a molecular weight exclusion size of from 5000 to 50,000 daltons, preferably of 10,000 daltons.

9. The process as claimed in claim 8, wherein the tissue factor is further purified from the retentate of the diafiltration.

10. The process as claimed in claim 9, wherein a multi-step chromatographic purification process is used.

11. The process as claimed in claim 10, wherein one of the chromatographic steps is an ion exchange chromatography on a cation exchanger or an anion exchanger.

12. The process as claimed in claim 11, wherein a strong anion exchange group, preferably DEAE, coupled to a pressure-resistant carrier material, preferably cellulose, is used.

13. The process as claimed in claim 12, wherein protease inhibitors are added to the buffer.

14. The process as claimed in claim 13, wherein an affinity chromatographic step is included.

15. The process as claimed in claim 14, wherein an antibody against tissue factor or a physiological ligand of tissue factor is used as the affinity ligand.

16. The process as claimed in claim 15, wherein a polyclonal or monoclonal antibody against tissue factor is covalently coupled to a pressure-resistant column material, preferably sepharose.

17. The process as claimed in claim 16, wherein the tissue factor is removed from the affinity ligand by using salt, buffer having a defined pH value or chaotropic agents, preferably by using dilute glycine buffer having an acid pH, particularly preferably using 50 mM glycine pH 2.

18. The process as claimed in claim 10, wherein the chromatographically purified tissue factor is concentrated to a concentration of > 1 g/l using a diafiltration method.

## Revendications

1. Procédé pour la production de facteur tissulaire recombinant fonctionnel dans *Escherichia coli*, **caractérisé en ce que**
a) pour la culture des organismes procaryotes on utilise un milieu qui contient au moins les composants suivants : extrait de levure et hydrolysats peptiques et tryptiques de tissus animaux, et
b) la dérépression du gène recombinant s'effectue dans la phase stationnaire de croissance des bactéries, en présence d'une source de carbone, cette source de carbone étant consommée au cours de la phase initiale de la dérépression, et les cellules restant ensuite pendant une durée définie, mais d'au moins 1,5 heures, à l'état de carence en carbone, et
c) pendant l'expression les cellules sont ensuite alimentées en continu avec la source de carbone, avec apport maximum d'oxygène.

2. Procédé selon la revendication 1, **caractérisé en ce qu'**au moins une protéase de cet organisme hôte est génétiquement inactivée.

3. Procédé selon la revendication 1, **caractérisé en ce qu'**on utilise un vecteur plasmidique qui contient l'information génétique de l'ADNc humain.

4. Procédé selon la revendication 3, **caractérisé en ce que** ce vecteur plasmidique contient un promoteur inductible ou déréprimable ou une séquence signal spécifique qui transporte le produit de traduction primaire dans un compartiment cellulaire primaire.

5. Procédé selon la revendication 1, **caractérisé en ce que** pour la culture des organismes procaryotes on utilise un milieu qui contient au moins les composants suivants : extrait de levure, extrait de tryptone, NaCl, extrait de malt et sels tampon.

6. Procédé selon la revendication 5, **caractérisé en ce que** les cellules ainsi obtenues sont désintégrées par homogénéisation et ensuite mises à incuber en présence d'un détergent.

7. Procédé selon la revendication 6, **caractérisé en ce qu'**à l'extrait contenant un détergent on ajoute du polyéthylèneglycol, de préférence du PEG 600, et l'ajuste ainsi à une concentration de 0 à 40 %, de préférence de 1 %, et, après incubation à une température de 0 à 25 °C, de préférence de 4 °C, pendant 0 à 6 heures, de préférence une heure, on filtre pour obtenir un filtrat limpide, de préférence par filtration en flux tangentiel.

8. Procédé selon la revendication 7, **caractérisé en ce que** le filtrat clarifié est en outre traité par diafiltration sur des membranes, seuil de coupure de masse moléculaire 5 000 à 50 000 daltons, de préférence 10 000 daltons.

9. Procédé selon la revendication 8, **caractérisé en ce que** le facteur tissulaire provenant du rétentat de la diafiltration est purifié davantage.

10. Procédé selon la revendication 9, **caractérisé en ce qu'**on utilise un procédé de purification par chromatographie en plusieurs étapes.

11. Procédé selon la revendication 10, **caractérisé en ce que** l'une des étapes de chromatographie est une chromatographie par échange d'ions sur un échangeur de cations ou un échangeur d'anions.

12. Procédé selon la revendication 11, **caractérisé en ce qu'**on utilise un groupe fortement échangeur d'anions, le DEAE de préférence, couplé à une matière de support solide, la cellulose de préférence.

13. Procédé selon la revendication 12, **caractérisé en ce qu'**on ajoute au tampon des inhibiteurs de protéase.

14. Procédé selon la revendication 13, **caractérisé en ce qu'**une étape de chromatographie par affinité est incluse.

15. Procédé selon la revendication 14, **caractérisé en ce qu'**on utilise comme ligand d'affinité un anticorps dirigé contre le facteur tissulaire ou un ligand physiologique de facteur tissulaire.

16. Procédé selon la revendication 15, **caractérisé en ce qu'**un anticorps monoclonal ou polyclonal dirigé contre le facteur tissulaire est couplé par liaison covalente à une matière de garnissage de la colonne, résistant à la pression, le Sepharose par exemple.

17. Procédé selon la revendication 16, **caractérisé en ce qu'**on sépare le facteur tissulaire d'avec le ligand d'affinité en utilisant un sel, un tampon ayant un pH détermine ou des agents chaotropes, de préférence au moyen d'un tampon glycine dilué à pH acide, de façon particulièrement préférée avec de la glycine 50 mM, pH 2.

18. Procédé selon la revendication 10, **caractérisé en ce que** le facteur tissulaire purifié par chromatographie est concentré jusqu'à une concentration de > 1 g/l par un procédé de diafiltration.
